(19) Europäisches Patentamt
European Patent Office
Office européen des brevets

(11) Veröffentlichungsnummer: **0 177 826**
**A2**

# (12) EUROPÄISCHE PATENTANMELDUNG

(21) Anmeldenummer: **85112042.8**

(22) Anmeldetag: **23.09.85**

(51) Int. Cl.⁴: **A 61 K 37/43**, G 01 N 33/74

(30) Priorität: **06.10.84 DE 3436819**

(43) Veröffentlichungstag der Anmeldung: **16.04.86**
**Patentblatt 86/16**

(84) Benannte Vertragsstaaten: **AT BE CH DE FR GB IT LI LU NL SE**

(71) Anmelder: **HOECHST AKTIENGESELLSCHAFT,**
**Postfach 80 03 20, D-6230 Frankfurt am Main 80 (DE)**

(72) Erfinder: **Engels, Joachim, Prof. Dr., Feldbergstrasse 1, D-6242 Kronberg/Taunus (DE)**
Erfinder: **Uhlmann, Eugen, Dr., Zum Talblick 31, D-6246 Glashütten/Taunus (DE)**
Erfinder: **Wetekam, Waldemar, Dr., Zeilring 40/I, D-6239 Eppstein/Taunus (DE)**
Erfinder: **König, Wolfgang, Dr., Eppsteiner Strasse 25, D-6238 Hofheim am Taunus (DE)**
Erfinder: **Müllner, Hubert, Dr., Pestalozzistrasse 4, D-6233 Kelkheim (Taunus) (DE)**

(54) **Arzneimittel und Diagnostikum mit Wachstumshormon-Releasingfaktor-Wirkung.**

(57) Derivate des GRF (Growth Hormone Releasing Factor) und seiner Analoga, die am Carboxyterminus einen Glycinrest tragen, zeigen ebenfalls GRF-Wirkung und eignen sich deshalb als Arzneimittel, insbesondere zur Behandlung von Wachstumsstörungen.

HOECHST AKTIENGESELLSCHAFT HOE 84/F 237 Dr.KL/St

Arzneimittel mit GRF-Wirkung

BEZEICHNUNG GEÄNDERT siehe Titelseite

GRF (Growth Hormone Releasing Factor) wird im Hypothalamus gebildet und stimuliert in der Hypophyse die Sekretion des Wachstumshormons. Im natürlichen GRF liegt die carboxyterminale Aminosäure Leucin in Form des Carbonsäureamids vor. Solche Verbindungen sind auf gentechnologischem Wege nicht direkt zugänglich. Es wurde deshalb ein Verfahren zur gentechnologischen Synthese von GRF und ähnlichen Verbindungen vorgeschlagen, bei dem man ein Polypeptid der Formel

$$Y - R - NH - CH_2 - COOH$$

in der Y den Methioninrest oder einen über Methionin gebundenen Rest eines Bakterienproteins und R eine Peptidsequenz aus genetisch kodierbaren Aminosäuren bedeutet, erzeugt und dieses Produkt enzymatisch in das Polypeptid der Formel

$$Y - R - NH_2$$

umwandelt (nicht vorveröffentlichte Deutsche Offenlegungsschrift 3 327 007 bzw. Südafrikanisches Patent 84/5779). Die enzymatische Umwandlung des Glycylrestes in die Aminogruppe der Carbonamidfunktion ist an sich bekannt (A.F. Bradbury et al., Nature 298 (1982) 686-688; I. Husain et al., FEBS Letters 152 (1983) 277-281).

Es wurde nun gefunden, daß Verbindungen der Formel I

$$R' - NH - CH_2 - COOH \qquad (I)$$

in der R' die Aminosäuresequenz des GRF und analoger Peptide mit GRF-Wirkung bedeutet, GRF-Wirkung zeigen und somit unmittelbar als Arzneimittel geeignet sind. Die

Erfindung betrifft somit Verbindungen der Formel I zur Anwendung als Arzneimittel bzw. Diagnostikum. Andere Aspekte und bevorzugte Ausgestaltungen der Erfindung werden im folgenden näher erläutert bzw. in den Patentansprüchen niedergelegt.

Eine Verbindung der Formel I, in der R' von der Aminosäuresequenz des natürlichen GRF abweicht, besteht darin, daß in Position 27 Met durch Leu ersetzt ist. Diese Verbindung und ihre Herstellung ist in der Deutschen Offenlegungsschrift 3 327 007 eingehend beschrieben. Ähnliche Verbindungen können analog hergestellt werden.

Die Wirkung des GRF besteht - definitionsgemäß - darin, daß er die Ausschüttung des Wachstumshormons bewirkt. GRF kann deshalb als Diagnostikum dafür dienen, ob das Wachstumshormon freigesetzt wird. Andererseits dient GRF als Heilmittel bei Wachstumsstörungen, also zur Steigerung des Wachstum bei Kindern und Jugendlichen. Darüberhinaus zeigt GRF eine Reihe von weiteren Wirkungen, die seinen Einsatz bei der Behandlung von Krankheiten Erwachsener erlauben: Es fördert die Wund- und Knochenbruchheilung, bewirkt einen Aufbau von Eiweiß bei konsumierenden Prozessen mit starker Eiweißeinschmelzung und hemmt bei gleichmäßig hoher Dosierung die Sekretion des Wachstumshormons, was die Behandlung von Hypophysenadenomen erlaubt und so gegen Akromegalie wirkt.

Da humaner GRF nicht artspezifisch wirkt, können Verbindungen der Formel I auch in der Veterinärmedizin bzw. zur Wachstumsbeschleunigung in der Tierzucht und zur Steigerung der Milchleistung eingesetzt werden.

Die Verbindungen der Formel I zeigen im Organismus eine GRF-Wirkung (Freisetzung des Wachstumshormons), die in quantitativer Beziehung der des natürlichen GRF bzw. der

entsprechenden Analoga weitgehend entspricht. Die in der Human- und/oder Veterinärmedizin anzuwendende Dosis entspricht somit auch der jeweils für GRF bzw. dessen Analoga unter Berücksichtigung der spezifischen biologischen Wirkstärke dieser Verbindungen anzuwendenden Dosis. Auch die möglichen Darreichungsformen entsprechen denen von GRF und dessen Derivaten, wobei insbesondere die nasale Anwendung von Bedeutung ist, da hier etwa 1 - 2 % der zugeführten Dosis zur Wirkung gelangen, womit eine Dauertherapie möglich wird. Auch die Anwendung in Form von Depotzubereitungen als Reservoir- oder Matrixsystem (Deutsche Offenlegungsschrift 3 336 197 bzw. veröffentlichte Europäische Patentanmeldung 0 133 988) und als biodegradierbare mikroverkapselte Arzneiform (beispielsweise Deutsche Patentanmeldung P 34 28 372.2) wird durch die gentechnische Herstellung der Verbindungen der Formel I ermöglicht.

PATENTANSPRÜCHE:

1. Verbindungen der Formel I

$$R' - NH - CH_2 - COOH \qquad (I)$$

in der R' die Aminosäuresequenz von GRF oder eines analogen Peptids mit GRF-Wirkung bedeutet, zur Anwendung als Arzneimittel oder Diagnostikum.

2. Verbindungen der Formel I zur Behandlung von Wachstumsstörungen.

3. Verwendung von Verbindungen der Formel I zur Herstellung eines Arzneimittels für die Behandlung von Wachstumsstörungen.

4. Verwendung von Verbindungen der Formel I zur Herstellung eines Diagnostikums.

5. Arzneimittel, gekennzeichnet durch einen Gehalt an einer Verbindung der Formel I.

6. Diagnostikum, gekennzeichnet durch einen Gehalt an einer Verbindung der Formel I.

PATENTANSPRÜCHE für Österreich:

1. Verwendung einer Verbindung der Formel I

$$R' - NH - CH_2 - COOH \qquad (I)$$

in der R' die Aminosäuresequenz von GRF oder eines analogen Peptids mit GRF-Wirkung bedeutet, zur Herstellung einer pharmazeutischen Zubereitung für die Behandlung oder zur Diagnose von Wachstumsstörungen.

2. Verwendung einer Verbindung der Formel I

$$R' - NH - CH_2 - COOH \qquad (I)$$

in der R' die Aminosäuresequenz von GRF oder eines analogen Peptids mit GRF-Wirkung bedeutet, zur Herstellung eines Arzneimittels für die Behandlung von Wachstumsstörungen.

3. Verfahren zur Herstellung eines Arzneimittels oder Diagnostikums, dadurch gekennzeichnet, daß man eine Verbindung der Formel I

$$R' - NH - CH_2 - COOH \qquad (I)$$

in der R' die Aminosäuresequenz von GRF oder eines analogen Peptids mit GRF-Wirkung bedeutet, in eine zur Darreichung geeignete Form bringt.